# EUROPÄISCHE PATENTSCHRIFT

(11) **EP 2 263 642 B1**
(45) Veröffentlichungstag und Bekanntmachung des Hinweises auf die Patenterteilung: **15.04.2020**
(21) Anmeldenummer: 10161107.7
(22) Anmeldetag: 27.04.2010
(51) Int. Cl.: A61K 8/38, A61K 8/49, A61K 8/55, A61Q 5/06, A61Q 5/08, A61K 8/25, A61K 8/31, A61K 8/19, A61K 8/22

(54) **Färbende Blondierungen**
Colouring bleaches
Décoloration colorante

(30) Priorität: 16.06.2009 DE 102009026967
(43) Veröffentlichungstag der Anmeldung: 22.12.2010
(73) Patentinhaber: Henkel AG & Co. KGaA, 40589 Düsseldorf (DE)
(72) Erfinder: Manneck, Hartmut, 23860, Klein Wesenberg (DE)

(56) Entgegenhaltungen:
- EP-A1- 1 666 095
- EP-A1- 1 738 801
- EP-A1- 1 759 684
- WO-A1-02/074270
- WO-A1-2010/015441
- DE-A1- 2 624 690
- DE-A1- 19 745 293
- DE-U1- 20 303 559

## Beschreibung

Gegenstand der vorliegenden Anmeldung sind Verfahren zur Aufhellung und zur Mattierung von Blondierungen keratinischer Fasern, insbesondere menschlicher Haare. Diese Verfahren ermöglichen ein Blondieren keratinischer Fasern ohne unerwünschte Farbverschiebung in gelbe bis rötliche Nuancen, und zeichnen sich durch eine exzellente Stabilität der eingesetzten Farbstoffe aus. Dazu werden die Mittel durch Vermischen von einer Oxidationszubereitung, einem Blondierpulver und einer Färbezubereitung, hergestellt, wobei die Färbezubereitung einen reduzierten pH-Wert besitzt. Weiterhin werden eine Verwendung zur Blondierung menschlicher Haare ohne unerwünschte Farbverschiebung sowie eine Mehrkomponentenverpackungseinheit, die die Einzelkomponenten dieser Verfahren umfasst, beschrieben.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Die Grundlagen der Blondierverfahren sind dem Fachmann bekannt und in einschlägigen Monographien, z. B. von K. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, zusammenfassend beschrieben.

Zum Blondieren menschlicher Haare werden üblicherweise feste oder pastenförmige Zubereitungen mit festen Oxidationsmitteln unmittelbar vor der Anwendung mit einer verdünnten Wasserstoffperoxid-Lösung vermischt. Diese Mischung wird dann auf das Haar aufgebracht und nach einer bestimmten Einwirkzeit wieder ausgespült. Die Einwirkungsdauer auf dem Haar zur Erzielung einer vollständigen Ausfärbung Aufhellung liegt zwischen etwa 30 und 60 Minuten. Es ist naheliegend, dass bei den Benutzern dieser Blondiermittel ein Bedürfnis besteht, diese Einwirkzeit zu verringern. Um eine ausreichenden Blondierwirkung zu erzielen, sind derartige Mittel üblicherweise stark alkalisch eingestellt, der pH-Wert liegt dabei zwischen 9 und 10,5. Derart hohe pH-Werte sind erforderlich, um eine Öffnung der äußeren Schuppenschicht (Cuticula) zu gewährleisten und somit eine Penetration der aktiven Spezies (Wasserstoffperoxid) ins Haar zu ermöglichen.

Um beim Bleichvorgang große Farbunterschiede zu erzielen, also insbesondere dunkle oder schwarze Haare zu entfärben, werden neben den bereits erwähnten Wasserstoffperoxidzubereitungen zumeist Bleichaktivatoren wie beispielsweise Persalze als zusätzliche Peroxidquellen, und/oder Carbonate als zusätzliche Alkalisierungsmittel benötigt. Somit stellt die Blondierung von Haaren mit dunkler Ausgangsfarbe eine besondere Herausforderung dar. Die natürliche Haarfarbe wird durch Melamine in der Cortex der Haarfaser bestimmt, wobei das Verhältnis von der zwei Pigmentklassen, Eumelanine mit bräunlich-schwarzen Tönen und Pheomelanine mit rötlichorangen Tönen, die eigentliche Haarfarbe bestimmt. Üblicherweise werden beim Blondierprozess die natürlichen Melanin-Farbstoffe durch oxidative Einwirkung zerstört und so eine Entfärbung der Fasern erreicht. Aufgrund der unterschiedlichen oxidativen Zerstörungsrate der verschiedenen Melaninpigmentklassen werden Haare jedoch nicht gleichmäßig entfärbt. In dunkleren Fasern mit hohem Melaningehalt verbleibt zumeist ein bestimmter Anteil an Farbstoffen, der sich häufig in gelblichen bis rötlichen Nuancen niederschlägt. Daher kommt es insbesondere beim Blondieren von dunkleren Haaren zu einer Farbverschiebung in Richtung warmer Töne.

Üblicherweise sind solche Farbverschiebungen in Richtung warmer Töne bei dem Anwender unerwünscht. Daher wird dieser Farbverschiebung zumeist durch eine Tönung mit der entsprechenden Komplementärfarbe gemäß der Farbenlehre entgegengewirkt. Ziel ist dabei ein silbrig-kühler Eindruck des Bleichergebnisses. Der Fachmann spricht in diesem Zusammenhang von einer Mattierung. Je nach Ausgangshaarfarbe muss dabei eine angepasste Tönungsmittelmischung eingesetzt werden, um entsprechend rötlichere Farbverschiebungen durch grünlichere Tönungsmittel oder gelblichere Farbverschiebungen durch eher violette Tönungsmittel auszugleichen.

Unter den aggressiven Bedingungen (starke Oxidationsmittel, hoher pH-Wert) der aufhellenden Zubereitung sowie der den im Stand der Technik üblichen Cremezubereitungen (meist pH > 11) sind die eingesetzten Tönungsmittel zumeist nicht ausreichend stabil. Daher müssen Blondierschritt und Tönungsschritt üblicherweise nacheinander erfolgen. Aus Gründen des Anwendungskomforts ist aber eine einstufige Blondierung, die bereits den mattierenden Farbausgleich der unerwünschten Warmtöne beinhaltet, besonders vorteilhaft. Daher besteht ein Bedürfnis nach Blondiermitteln, die durch Zusatz von stabilen Tönungskomponenten in die Anwendungszubereitung einen guten Farbausgleich und damit eine hohe Mattierung erzielen.

EP 1759684 A1 offenbart ein Verfahren zur gleichzeitigen Färbung und Bleichung von Keratinfasern, wobei direktziehende Farbstoffe eingesetzt werden, die gegenüber Bleichmitteln der Kategorie 2 stabil sind. EP 1666095 A1 beschreibt Zusammensetzungen zur gleichzeitigen Färbung und Bleichung von Keratinfasern, enthaltend bestimmte meta-substituierte ortho-Nitroaniline. Gegenstand der DE 20303559 U1 sind Zusammensetzungen zur gleichzeitigen Färbung von Bleichung von Keratinfasern, enthaltend gegen Peroxidsalze stabile Farbstoffe aus diversen chemischen Farbstoffklassen. WO 02/074270 A1 betrifft Zusammensetzungen zur gleichzeitigen Färbung und Bleichung von Keratinfasern, wobei direktziehende Farbstoffe eingesetzt werden, die gegen Bleichmittel der Kategorie 2 stabil sind.

Im Rahmen ihrer Untersuchungen hat die Anmelderin nun überraschend gefunden, dass sich direktziehende Farbstoffe hervorragend in einer Färbezubereitung stabilisieren lassen, wenn es sich um halogenierte, direktziehende Farbstoff handelt, und wenn die Färbezubereitung einen reduzierten pH-Wert gegenüber dem Stand der Technik aufweist.

Ein erster Gegenstand der vorliegenden Erfindung ist daher ein Verfahren, bei dem ein fließfähiges Mittel zur Aufhellung und Färbung menschlicher Haare durch Vermischen hergestellt wird, auf menschliches Haar aufgetragen wird, das Mittel für einen Zeitraum von 2 bis 60 min im Haar belassen wird, und das Haar anschließend mit Wasser und/oder einem handelsüblichen Shampoo gespült wird, dadurch gekennzeichnet, dass das Mittel durch Vermischen von
i. mindestens einer Oxidationsmittelzubereitung (A), enthaltend in einem flüssigen, kosmetischen Träger mindestens ein Oxidationsmittel,
ii. mindestens einem Blondierpulver (B), enthaltend mindestens einen Bleichkraftverstärker und mindestens ein festes Alkalisierungsmittel, und
iii. mindestens einer Färbezubereitung (C), enthaltend in einem flüssigen, kosmetischen Träger mindestens einen halogenierten, direktziehenden Farbstoff, ausgewählt aus der Gruppe, die gebildet wird aus Tetrabromphenolblau und Acid Red 92, und zusätzlich mindestens einen Komplexbildner, bei einem pH-Wert von kleiner 10, hergestellt wird,
dadurch gekennzeichnet, dass die Gesamtmenge an direktziehenden Farbstoffen 0,0001 bis 2,5 Gew.-%, bezogen auf das Gesamtgewicht des anwendungsbereiten Mittels, beträgt,
wobei sich die Angaben "fließfähig", "fest" und "flüssig" auf eine Temperatur im Bereich zwischen 15 und 40 °C beziehen.

Obwohl die erfindungsgemäßen Verfahren in erster Linie zum Aufhellen und Färben von menschlichen Haaren geeignet sind, steht prinzipiell einer Verwendung auch auf anderen keratinhaltigen bzw. keratinischen Fasern, wie Pelze, Wolle, Federn, sowie auf anderen Gebieten nichts entgegen.

Im durch das erfindungsgemäße Verfahren hergestellten Mittel liegen die Wirkstoffe der Oxidationsmittelzubereitung (A) und der Färbezubereitung (C) in einem flüssigen, kosmetischen Träger vor. Dieser kosmetische Träger ist im Sinne der Erfindung ist wässrig, alkoholisch oder wässrig-alkoholisch. Zum Zwecke der Haarbehandlung sind solche Träger zumeist Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Ein wässriger Träger enthält im Sinne der Erfindung mindestens 30 Gew.-%, insbesondere mindestens 50 Gew.-% Wasser, bezogen auf das Gesamtgewicht der Anwendungsmischung.

Die im erfindungsgemäßen Verfahren hergestellten Mittel werden durch Vermischen einer Oxidationszubereitung (A) mit einem Blondierpulver (B) und einer Färbezubereitung (C) hergestellt. Dabei werden die anwendungsbereiten Mittel bevorzugt zeitnah zur Anwendung, besonders bevorzugt unmittelbar vor der Anwendung hergestellt. Die einzelnen Komponenten werden entweder in gleichen Gewichtsteilen zueinander oder mit nur einem geringen Überschuss untereinander eingesetzt. Eine geeignete Ausführungsform ist auch dadurch gekennzeichnet, dass die Oxidationszubereitung (A) im Überschuss zu den anderen Komponenten (B) und (C) vorliegt. Eine besondere Ausführungsform ist dadurch gekennzeichnet, dass das anwendungsbereite Mittel aus einem Gewichtsteil Oxidationszubereitung (A) und einem Gewichtsteil, bestehend aus Komponente (B) und (C), hergestellt wird.

Es kann dabei unerheblich sein, ob zunächst eine Mischung aus (A) und (B) hergestellt wird, und anschließend die Färbezubereitung (C) zugemischt wird, oder ob eine davon verschiedene Reihenfolge der Vermischung der einzelnen Komponenten genutzt wird.

Die anwendungsbereiten Mittel sind dabei fließfähig. Unter fließfähig wird dabei eine Zubereitung verstanden, welche bei Raumtemperatur eine solche Rheologie aufweist, dass sie mit den Händen oder einem üblichen Hilfsmittel wie einer Bürste auf dem Haar verteilbar ist.

Erster Bestandteil des anwendungsbereiten Mittels ist eine Oxidationsmittelzubereitung (A).

Die Oxidationsmittelzubereitung (A) enthält mindestens ein Oxidationsmittel. Erfindungsgemäß geeignete Oxidationsmittel sind ausgewählt aus Wasserstoffperoxid und dessen Anlagerungsverbindungen an feste Träger.

Als erfindungsgemäßes Oxidationsmittel wird bevorzugt Wasserstoffperoxid selbst verwendet. Das Wasserstoffperoxid wird als Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidinon n H₂O₂ (n ist eine positive ganze Zahl größer 0), Harnstoffperoxid und Melaminperoxid, eingesetzt.

Erfindungsgemäß bevorzugte Oxidationszubereitungen (A) sind wässrige WasserstoffperoxidLösungen. Die Konzentration einer Wasserstoffperoxid-Lösung wird einerseits von den gesetzlichen Vorgaben und andererseits von dem gewünschten Effekt bestimmt. Vorzugsweise werden 3 Gew.-%ige bis 12 Gew.-%ige Lösungen in Wasser verwendet. Bevorzugt sind erfindungsgemäße Mittel zur Blondierung menschlicher Haare, die 0,5 bis 18 Gew.-%, vorzugsweise 1 bis 15 Gew.-%, besonders bevorzugt 2,5 bis 12 Gew.-% und insbesondere 3 bis 9 Gew.-% Wasserstoffperoxid, jeweils berechnet als 100%iges H₂O₂ und bezogen auf das Gesamtgewicht des anwendungsbereiten Mittels, enthalten.

Erfindungsgemäße Oxidationsmittelzubereitungen (A) sind vorzugsweise wässrige, fließfähige Oxidationsmittelzubereitungen. Dabei sind bevorzugte Zubereitungen dadurch gekennzeichnet, dass die fließfähige Oxidationsmittelzubereitung - bezogen auf ihr Gewicht - 40 bis 98 Gew.-%, vorzugsweise 60 bis 97,5 Gew.-%, besonders bevorzugt 70 bis 97 Gew.-%, weiter bevorzugt 75 bis 96 Gew.-% und insbesondere 80 bis 95 Gew.-% Wasser enthält.

Erfindungsgemäß kann aber die Oxidationsmittelzubereitung auch zusammen mit einem Katalysator auf das Haar aufgebracht werden. Solche Katalysatoren sind z. B. bestimmte Enzyme, lodide, Chinone oder Metallionen.

Geeignete Enzyme sind z. B. Peroxidasen, die die Wirkung geringer Mengen an Wasserstoffperoxid deutlich verstärken können. Ein Einsatz bestimmter Metallionen oder -komplexe kann ebenfalls bevorzugt sein. Geeignete Metallionen sind beispielsweise Zn²⁺, Cu²⁺, Fe²⁺, Fe³⁺, Mn²⁺, Mn⁴⁺, Li⁺, Mg²⁺, Ca²⁺, Ce⁴⁺, V³⁺, Co²⁺, Ru³⁺ und Al³⁺, besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate, Citrate und Tartrate. Durch Verwendung dieser Metallsalze kann die Blondierwirkung gezielt beeinflusst werden. Besonders bevorzugte Mittel enthalten diese Metallionen zu 0,0001 bis 2,5 Gew.-%, vorzugsweise 0,001 bis 1 Gew.-%, bezogen auf das Gesamtgewicht des erfindungsgemäßen Mittels.

Weiterhin hat es sich als vorteilhaft erweisen, wenn die Oxidationsmittelzubereitungen mindestens einen Stabilisator oder Komplexbildner enthalten. Besonders bevorzugte Stabilisatoren sind Phenacetin, Alkalibenzoate (Natriumbenzoat) und Salicylsäure.

Erfindungsgemäß bevorzugt ist auch der Einsatz von Komplexbildnern. Komplexbilder sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt, d. h. mindestens "zweizähnig" ist. Gebräuchliche und im Rahmen der vorliegenden Erfindung können alle Komplexbildner des Standes der Technik eingesetzt werden. Diese können unterschiedlichen chemischen Gruppen angehören. Vorzugsweise werden sie einzeln oder im Gemisch miteinander eingesetzt:
a) Polycarbonsäuren, bei denen die Summe der Carboxyl- und gegebenenfalls Hydroxylgruppen mindestens 5 beträgt wie Gluconsäure;
b) stickstoffhaltige Mono- oder Polycarbonsäuren wie Ethylendiamintetraessigsäure (EDTA), N-Hydroxyethylethylendiamintriessigsäure, Diethylentriaminpentaessigsäure (DTPA), Ethylendiamindibernsteinsäure (EDDS), Hydroxyethyliminodiessigsäure, Nitridodiessigsäure-3-propionsäure, Isoserindiessigsäure, N,N-Di-(2-hydroxyethyl)glycin, N-(1,2-Dicarboxy-2-hydroxyethyl)glycin, N-(1,2-Dicarboxy-2-hydroxyethyl)asparaginsäure oder Nitrilotriessigsäure (NTA), Ethylendiamindiglutarsäure (EDGA), 2-Hydroxypropylendiamindibernsteinsäure (HPDS), Glycinamid-N,N'-dibernsteinsäure (GADS), Ethylendiamin-N-N'-diglutarsäure (EDDG), 2-Hydroxypropylendiamin-N-N'-dibernsteinsäure (HPDDS), Diaminoalkyldi-(sulfobernsteinsäure) (DDS), Ethylendicysteinsäure (EDC), Ethylendiamin-N-N'-bis(orthohydroxyphenyl)essigsäure (EDDHA), N-2-Hydroxyethylamin-N,N-diessigsäure, Glyceryliminodiessigsäure, Iminodiessigsäure-N-2-hydroxypropylsulfonsäure, Asparaginsäure-N-carboxymethyl-N-2,5-hydroxypropyl-3-sulfonsäure,β-Alanin-N,N'-diessigsäure, Asparaginsäure-N,N'-diessigsäure, Asparaginsäure-N-monoessigsäure, Dipicolinsäure, sowie deren Salze und/oder Derivate;
c) geminale Diphosphonsäuren wie 1-Hydroxyethan-1,1-diphosphonsäure (HEDP), deren höhere Homologe mit bis zu 8 Kohlenstoffatomen sowie Hydroxy- oder Aminogruppen-haltige Derivate hiervon und 1-Aminoethan-1,1-diphosphonsäure, deren höhere Homologe mit bis zu 8 Kohlenstoffatomen sowie Hydroxy- oder Aminogruppen-haltige Derivate;
d) Aminophosphonsäuren wie Ethylendiamintetra(methylenphosphonsäure) (EDTMP), Diethylen-triaminpenta(methylenphosphonsäure) (DTPMP) sowie deren höhere Homologe, oder Nitrilotri(methylenphosphonsäure);
e) Phosphonopolycarbonsäuren wie 2-Phosphonobutan-1,2,4-tricarbonsäure sowie
f) Cyclodextrine,
g) Alkalistannate (Natriumstannat), Alkalipyrophosphate (Tetranatriumpyrophosphat, Dinatriumpyrophosphat), Alkaliphosphate (Natriumphosphat), und Phosphorsäure.

Bei den erfindungsgemäß erforderlichen alkalischen pH-Werten der Behandlungslösungen liegen diese Komplexbildner zumindest teilweise als Anionen vor. Es ist unwesentlich, ob sie in Form der Säuren oder in Form von Salzen eingebracht werden. Im Falle des Einsatzes als Salze sind Alkali-, Ammonium- oder Alkylammoniumsalze, insbesondere Natriumsalze, bevorzugt. Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Die Oxidationsmittelzubereitung (A) enthält neben dem eigentlichen Oxidationsmittel gegebenenfalls weitere Hilfs- und Zusatzstoffe. So hat es sich erfindungsgemäß als bevorzugt erwiesen, wenn die Oxidationsmittelzubereitung mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Gemäß einer ersten bevorzugten Ausführungsform handelt es sich bei dem Verdickungsmittel um ein anionisches, synthetisches Polymer. Solche (Co-)Polymere leiten sich zumindest von einem anionischen Monomer ab. Beispiele für anionische Monomere sind Acrylsäure, Methacrylsäure, Crotonsäure, Itaconsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind Maleinsäureanhydrid sowie insbesondere 2-Acrylamido-2-methylpropansulfonsäure und Acrylsäure. Bevorzugte anionische Homopolymere sind unvernetzte und vernetzte Polyacrylsäuren. Geeignete anionische Homopolymere und Copolymere sind beispielsweise unter den Warenzeichnen Carbopol® , Aculyn® 33, Aculyn® 22, Structure® 2001, Structure® 3001, Carbopol® ETD 2020, Carbopol® Ultrez 20, Pemulen TR-1, Sepigel®305, Sirmulgel® 600, Aristoflex® AVC, Aristoflex® HMB, Stabileze® QM, Rheothik®11-80 oder Latekoll® D im Handel erhältlich.

Gemäß einer zweiten Ausführungsform handelt es sich bei dem Verdickungsmittel um einen kationisches synthetisches Polymer. Ein geeignetes Homopolymer ist das, gewünschtenfalls vernetzte, Poly(methacryloxyethyltrimethylammonium Chlorid) mit der INCI-Bezeichnung Polyquaternium-37 (Salcare® SC 92, Salcare® SC 95 und Salcare® SC 96).

In einer dritten bevorzugten Ausführungsform werden natürlich vorkommende Verdickungsmittel eingesetzt. Erfindungsgemäß können sowohl modifizierte als auch unmodifizierte Guargums zum Einsatz kommen, wobei Jaguar® C ein Beispiel für unmodifizierte Guargums ist und modifizierte Guargums unter den Handelsbezeichnungen Jaguar® HP8, Jaguar® HP60, Jaguar® HP120, Jaguar® DC 293 und Jaguar® HP105 erhältlich sind.

Weiterhin geeignete natürliche Verdickungsmittel sind Biosaccharidgums mikrobiellen Ursprungs, wie die Skleroglucangums oder Xanthangums, Gums aus pflanzlichen Exsudaten, wie Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, Cellulosederivate, wie beispielsweise Methylcellulose, Carboxyalkylcellulosen und Hydroxyalkylcellulosen (Handelsnamen Cellosize®, Natrosol®, Blanose®, Aquasorb®, Ambergum® und Cellgon®). Bevorzugt sind weiterhin Stärke und deren Derivate, beispielsweise aus Kartoffeln, Maniok, Bataten, Maranta, Mais, Getreide, Reis, Hülsenfrüchten, Bananen oder dem Mark der Sagopalme gewonnen werden.

Auch nichtionische, vollsynthetische Polymere, wie Polyvinylalkohol oder Polyvinylpyrrolidinon, sind als erfindungsgemäße Verdickungsmittel einsetzbar (Luviskol®).

Als anorganische Verdickungsmittel haben sich Schichtsilikate (polymere, kristalline Natriumdisilicate) als besonders geeignet im Sinne der vorliegenden Erfindung erwiesen. Insbesondere Tone, insbesondere Magnesium Aluminium Silicate, wie beispielsweise Bentonit, besonders Smektite, wie Montmorillonit oder Hectorit, und synthetische Schichtsilikate, wie Magnesiumschichtsilikat (Optigel®), sind bevorzugt.

Weiterer Bestandteil des erfindungsgemäßen, anwendungsbereiten Mittels ist mindestens ein Blondierpulver (B). Das Blondierpulver (B) enthält mindestens einen Bleichkraftverstärker und mindestens ein festes Alkalisierungsmittel.

Als Bleichkraftverstärker können im Rahmen dieser Erfindung Peroxoverbindungen, weiterhin Verbindungen, die unter Perhydrolysebedingungen aliphatische Peroxocarbonsäuren und/oder substituierte Perbenzoesäure ergeben, Kohlensäurederivate, Alkylcarbonate, -carbamate, Silylcarbonate, -carbamate und stickstoffhaltige, gegebenenfalls kationische Heterocyclen, insbesondere Acylpyridinium und Dihydroisochinolinium-Salze, eingesetzt werden.

Vorzugsweise ist der Bleichkraftverstärker ausgewählt aus Ammoniumpersulfat, Alkalimetallpersulfaten, Ammoniumperoxomonosulfat, Alkalimetallhydrogenperoxomonosulfaten, Alkalimetallperoxodiphosphaten und Erdalkalimetallperoxiden. Besonders bevorzugte Bleichkraftverstärker sind Ammoniumperoxodisulfat, Kaliumperoxodisulfat, Natriumperoxodisulfat, Kaliumhydrogenperoxomonosulfat, Kaliumperoxodiphosphat, Magnesiumperoxid und Bariumperoxid.

Erfindungsgemäß besonders bevorzugt sind Mittel, die in der Blondierzubereitung (B) als Bleichkraftverstärker mindestens ein anorganisches Salz, ausgewählt aus Peroxodisulfaten, enthalten. Weiterhin hat es sich bei den Arbeiten zur vorliegenden Erfindung als besonders bevorzugt erwiesen, wenn die erfindungsgemäßen Mittel mindestens zwei verschiedene Peroxodisulfate enthalten. Bevorzugte Peroxodisulfatsalze sind dabei Kombinationen aus Ammoniumperoxodisulfat und Kaliumperoxodisulfat und/oder Natriumperoxodisulfat.

Die Peroxoverbindungen sind in einer Menge von 0,1 bis 25 Gew.-%, insbesondere in einer Menge von 0,5 bis 15 Gew.-%, bezogen auf das Gesamtgewicht des anwendungsbereiten Mittels, enthalten.

Der Einsatz von Persulfatsalzen bzw. Peroxodisulfatsalzen erfolgt in der Regel in Form eines gegebenenfalls entstaubten Pulvers.

Die erfindungsgemäßen wasserfreien Zusammensetzungen anstelle und/oder zusätzlich zu den festen Peroxoverbindungen einen weiteren Bleichkraftverstärker enthalten. Als Bleichkraftverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxo-hexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. i-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Als Bleichverstärker vom Typ der Kohlensäurederivate können erfindungsgemäß bevorzugt Carbonatsalze bzw. Hydrogencarbonatsalze eingesetzt werden. Diese sind bevorzugt ausgewählt aus der Gruppe der Ammonium-, Alkalimetall- (insbesondere Natrium- und Kalium-) sowie Erdalkalimetall- (insbesondere Magnesium- und Calcium-), -carbonatsalze bzw. -hydrogencarbonatsalze.

Besonders bevorzugte Carbonat- bzw. Hydrogencarbonatsalze sind Ammoniumhydrogencarbonat, Ammoniumcarbonat, Natriumhydrogencarbonat, Natriumcarbonat, Kaliumhydrogencarbonat, Kaliumcarbonat, Magnesiumcarbonat und Calciumcarbonat. Diese besonders bevorzugten Salze können allein oder in deren Mischungen von mindestens zwei Vertretern als Bleichverstärker verwendet werden.

Als Bleichverstärker vom Typ der Monoalkylcarbonate und deren Derivate werden bevorzugt mindestens ein Kohlensäuremonoester und/oder mindestens ein Kohlensäuremonoamid in dem erfindungsgemäßen Verfahren verwendet. Das acide H-Atom des Kohlensäuremonoesters bzw. - monoamids kann auch in neutralisierter Form vorliegen, d.h. es können erfindungsgemäß auch Salze von Kohlensäuremonoestern bzw. Kohlensäuremonoamiden eingesetzt werden. Hier sind erfindungsgemäß Kohlensäuremonoester bzw. das Kohlensäuremonoamide bevorzugt, die in ganz oder teilweise neutralisierter Form, vorzugsweise in Form des Alkalimetall-, Ammonium-, Erdalkalimetall- oder Aluminiumsalzes und insbesondere in Form seines Natriumsalzes, vorliegen. Als Bleichverstärker vom Typ der Silylcarbonate und deren Derivate werden bevorzugt mindestens ein Silylcarbonat und/oder mindestens ein Silylcarbamat in die erfindungsgemäßen Zusammensetzungen eingearbeitet.

Weiterhin erfindungsgemäß einsetzbare Bleichkraftverstärker können aus stickstoffhaltigen, gegebenenfalls kationischen Heterocyclen. Als Beispiel eines stickstoffhaltigen heterocyclischen Bleichkraftverstärkers ist insbesondere Imidazol zu nennen. Bevorzugt werden stickstoffhaltige, heterocyclische Bleichkraftverstärker aber unter kationischen Heterocyclen gewählt. Hierbei sind insbesondere kationische Pyridine, Pyrimidine, Pyrazine, Pyridazine, Triazine, Imidazole, Thiazole, Oxazole, Isoxazole, Chinoline, Isochinoline sowie deren partielle oder vollständig gesättigten Analoga.

Besonders bevorzugte stickstoffhaltige, heterocyclische Bleichkraftverstärker sind die quaternierten Kationen von Pyridinen und 3,4-Dihydroisochinolinen.

Bevorzugte Verbindungen sind dabei 4-Acetyl-1-methylpyridinium-p-toluolsulfonat, 4-Acetyl-1-methylpyridiniumbenzolsulfonat, 4-Acetyl-1-methylpyridiniumbromid, 4-Acetyl-1-methylpyridinium-hydrogensulfat, 4-Acetyl-1-allylpyridinium-p-toluolsulfonat, 4-Acetyl-1-allylpyridiniumbenzol-sulfonat, 4-Acetyl-1-allylpyridiniumbromid, 4-Acetyl-1-allylpyridiniumhydrogensulfat, 4-Acetyl-1-(2-hydroxyethyl)pyridinium-p-toluolsulfonat, 4-Acetyl-1-(2-hydroxyethyl)pyridiniumbenzolsulfonat, 4-Acetyl-1-(2-hydroxyethyl)pyridiniumbromid, 4-Acetyl-1-(2-hydroxyethyl)pyridiniumhydrogensulfat, 4-Acetyl-1-(2-oxopropyl)pyridinium-p-toluolsulfonat, 4-Acetyl-1-(2-oxopropyl)pyridiniumbenzol-sulfonat, 4-Acetyl-1-(2-oxopropyl)pyridiniumbromid, 4-Acetyl-1-(2-oxopropyl)pyridiniumhydrogen-sulfat, 4-Acetyl-1-ethylpyridinium-p-toluolsulfonat, 4-Acetyl-1-ethylpyridiniumbenzolsulfonat, 4-Acetyl-1-ethylpyridiniumbromid, 4-Acetyl-1-ethylpyridiniumhydrogensulfat, 4-Acetyl-1-(2-methyl-prop-2-enyl)pyridinium-p-toluolsulfonat, 4-Acetyl-1-(2-methyl-prop-2-enyl)pyridiniumbenzol-sulfonat, 4-Acetyl-1-(2-methyl-prop-2-enyl)pyridiniumbromid, 4-Acetyl-1-(2-methyl-prop-2-enyl)-pyridiniumhydrogensulfat, 4-Acetyl-1-benzylpyridinium-p-toluolsulfonat, 4-Acetyl-1-benzyl-pyridiniumbenzolsulfonat, 4-Acetyl-1-benzylpyridiniumbromid, 4-Acetyl-1-benzylpyridinium-hydrogensulfat, 4-Acetyl-1-(2-methoxyethyl)pyridinium-p-toluolsulfonat, 4-Acetyl-1-(2-methoxyethyl)pyridiniumbenzolsulfonat, 4-Acetyl-1-(2-methoxyethyl)pyridiniumbromid oder 4-Acetyl-1-(2-methoxyethyl)pyridiniumhydrogensulfat sowie 2-Acetyl-1-methylpyridinium-p-toluolsulfonat, 2-Acetyl-1-methylpyridiniumbenzolsulfonat, 2-Acetyl-1-methylpyridiniumbromid, 2-Acetyl-1-methyl-pyridiniumhydrogensulfat, 2-Acetyl-1-allylpyridinium-p-toluolsulfonat, 2-Acetyl-1-allylpyridinium-benzolsulfonat, 2-Acetyl-1-allylpyridiniumbromid, 2-Acetyl-1-allylpyridiniumhydrogensulfat, 2-Acetyl-1-(2-hydroxyethyl)pyridinium-p-toluolsulfonat, 2-Acetyl-1-(2-hydroxyethyl)pyridiniumbenzol-sulfonat, 2-Acetyl-1-(2-hydroxyethyl)pyridiniumbromid, 2-Acetyl-1-(2-hydroxyethyl)pyridinium-hydrogensulfat, 2-Acetyl-1-(2-oxopropyl)pyridinium-p-toluolsulfonat, 2-Acetyl-1-(2-oxopropyl)-pyridiniumbenzolsulfonat, 2-Acetyl-1-(2-oxopropyl)pyridiniumbromid, 2-Acetyl-1-(2-oxopropyl)-pyridiniumhydrogensulfat, 2-Acetyl-1-ethylpyridinium-p-toluolsulfonat, 2-Acetyl-1-ethylpyridinium-benzolsulfonat, 2-Acetyl-1-ethylpyridiniumbromid, 2-Acetyl-1-ethylpyridiniumhydrogensulfat, 2-Acetyl-1-(2-methyl-prop-2-enyl)pyridinium-p-toluolsulfonat, 2-Acetyl-1-(2-methyl-prop-2-enyl)-pyridiniumbenzolsulfonat, 2-Acetyl-1-(2-methyl-prop-2-enyl)pyridiniumbromid, 2-Acetyl-1-(2-methyl-prop-2-enyl)pyridiniumhydrogensulfat, 2-Acetyl-1-benzylpyridinium-p-toluolsulfonat, 2-Acetyl-1-benzylpyridiniumbenzolsulfonat, 2-Acetyl-1-benzylpyridiniumbromid, 2-Acetyl-1-benzyl-pyridiniumhydrogensulfat, 2-Acetyl-1-(2-methoxyethyl)pyridinium-p-toluolsulfonat, 2-Acetyl-1-(2-methoxyethyl)pyridiniumbenzolsulfonat, 2-Acetyl-1-(2-methoxyethyl)pyridiniumbromid oder 2-Acetyl-1-(2-methoxyethyl)pyridiniumhydrogensulfat.

Bevorzugte Verbindungen sind dabei N-Methyl-3,4-dihydroisochinolinium-p-toluolsulfonat, N-Methyl-3,4-dihydroisochinoliniumbenzolsulfonat, N-Methyl-3,4-dihydroisochinoliniumhydrogen-sulfat, N-Allyl-3,4-dihydroisochinolinium-p-toluolsulfonat, N-Allyl-3,4-dihydroisochinoliniumbenzol-sulfonat, N-Allyl-3,4-dihydroisochinoliniumbromid, N-Allyl-3,4-dihydroisochinoliniumacetat, 3,4-Dihydro-2-(3-hydroxypropyl)isochinolinium-p-toluolsulfonat, 3,4-Dihydro-2-(3-hydroxypropyl)isochinoliniumbenzolsulfonat, 3,4-Dihydro-2-(3-hydroxypropyl)isochinoliniumbromid, 3,4-Dihydro-2-(3-hydroxypropyl)isochinoliniumacetat, 3,4-Dihydro-2-(2-hydroxyethyl)isochinolinium-p-toluolsulfonat, 3,4-Dihydro-2-(2-hydroxyethyl)isochinoliniumbenzolsulfonat, 3,4-Dihydro-2-(2-hydroxyethyl)isochinoliniumbromid oder 3,4-Dihydro-2-(2-hydroxyethyl)isochinoliniumacetat.

Besonders bevorzugt ist 4-Acetyl-1-methylpyridinium-p-toluolsulfonat als Bleichkraftverstärker.

Die neben oder anstelle von Peroxoverbindungen eingesetzten Bleichkraftverstärker sind in den erfindungsgemäßen, kosmetischen Mitteln bevorzugt in Mengen von 0,5 bis 30 Gew.-%, insbesondere in Mengen von 2 bis 20 Gew.-%, jeweils bezogen auf das Gesamtgewicht des anwendungsbereiten Mittels, enthalten.

Zur weiteren Steigerung der Aufhellleistung können der erfindungsgemäßen Zusammensetzung zusätzlich als Bleichverstärker mindestens eine gegebenenfalls hydratisierte SiO₂-Verbindung zugesetzt. Obwohl bereits geringe Mengen der gegebenenfalls hydratisierten SiO₂-Verbindungen die Aufhellleistung erhöhen, kann es erfindungsgemäß bevorzugt sein, die gegebenenfalls hydratisierten SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder.

Hinsichtlich der gegebenenfalls hydratisierten SiO₂-Verbindungen unterliegt die vorliegende Erfindung prinzipiell keinen Beschränkungen. Bevorzugt sind Kieselsäuren, deren Oligomeren und Polymeren sowie deren Salze, insbesondere Natriumsalze, und Wassergläser, die aus einem Silikat der Formel (SiO₂)ₙ(Na₂O)ₘ(K₂O)ₚ gebildet werden, wobei n steht für eine positive rationale Zahl und m und p stehen unabhängig voneinander für eine positive rationale Zahl oder für 0, mit den Maßgaben, dass mindestens einer der Parameter m oder p von 0 verschieden ist und das Verhältnis zwischen n und der Summe aus m und p zwischen 1:4 und 4:1 liegt. Auch Metasilicate, die sich gemäß vorstehender Formel durch das Verhältnis zwischen n und der Summe aus m und p von ≤ 1 auszeichnen und sich als kettenförmige polymere Strukturen des Anions [SiO₃]²⁻ auffassen lassen, können bevorzugt eingesetzt werden. Natriummetasilicat der Formel [NaSiO₃]ₓ, ist dabei besonders bevorzugt.

Erfindungsgemäß besonders bevorzugte Wassergläser werden unter den Bezeichnungen Ferrosil® 119, Natronwasserglas 40/42, Portil® A, Portil® AW, Portil® W und Britesil® C20 vertrieben.

Es hat sich erfindungsgemäß als bevorzugt erwiesen, wenn das Blondierpulver (B) wasserfrei formuliert ist. Wasserfrei im Sinne der vorliegenden Erfindung bedeutet einen Wassergehalt bezogen auf das Pulver (B) von weniger als 5 Gew.-%, insbesondere von weniger als 2 Gew.-%. Blondierzubereitungen, die weniger als 0,1 Gew.-% Wasser, enthalten können erfindungsgemäß ganz besonders bevorzugt sein.

Das Blondierpulver (B) ist weiterhin bevorzugt staubfrei formuliert.

Im Falle der Formulierung als staubfreies Pulver hat es sich als besonders bevorzugt erwiesen, wenn die Zubereitung (B) mindestens einen nicht hydroxylierten Fettsäureester mit einem Schmelzpunkt von höchstens 50°C, insbesondere von höchstens 30°C, und/oder mindestens eine C₁₀-C₃₀-Fettsäure mit mindestens einer zusätzlichen Hydroxygruppe und/oder ein Derivat davon, enthält.

Ester von nicht hydroxylierten C₆-C₃₀-Alkylmonocarbonäuren mit C₂-C₃₀-Monoalkoholen eignen sich erfindungsgemäß bevorzugt als Fettsäureester. Bevorzugt sind die Monoester der Fettsäuren mit Monoalkoholen mit 2 bis 24 C-Atomen sowie deren technische Mischungen. Erfindungsgemäß besonders bevorzugt sind Isopropylmyristat (Rilanit® IPM), Isononansäure-C₁₆-C₁₈-alkylester (Cetiol® SN), 2-Ethylhexylpalmitat (Cegesoft® 24), Stearinsäure-2-ethylhexylester (Cetiol® 868), Cetyloleat, Kokosfettalkohol-caprinat/-caprylat (Cetiol® LC), n-Butylstearat, Oleylerucat (Cetiol® J 600), Isopropylpalmitat (Rilanit® IPP), Oleyloleat (Cetiol®), Laurinsäurehexylester (Cetiol® A), Myristylmyristat (Cetiol® MM), Cetearylisononanoat (Cetiol® SN), Ölsäuredecylester (Cetiol® V).

Die Blondierpulver (B) enthalten weiterhin mindestens ein festes Alkalisierungsmittel. Verwendbare Alkalisierungsmittel werden gewählt aus anorganischen Salzen, insbesondere der Alkali- und Erdalkalimetalle, und festen, organischen Alkalisierungsmitteln, insbesondere basischen Aminosäuren.

Es hat sich aber im Rahmen der Untersuchungen zur vorliegenden Erfindung herausgestellt, dass erfindungsgemäß bevorzugte Blondierpulver (B) dadurch gekennzeichnet sind, dass sie ein festes, anorganisches Alkalisierungsmittel enthalten. Das erfindungsgemäße, anorganische Alkalisierungsmittel wird bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat sowie deren Mischungen.

Das Blondierpulver (B) enthält die festen Alkalisierungsmittel bevorzugt in Mengen von 0,2 bis 25 Gew.-%, insbesondere 0,5 bis 10 Gew.-%.

Dritter Bestandteil des im erfindungsgemäßen Verfahren hergestellen anwendungsbereiten Mittels ist mindestens ein Färbezubereitung (C). Die Färbezubereitung (C) enthält dabei mindestens einen halogenierten, direktziehenden Farbstoff und zusätzlich mindestens einen Komplexbildner, und besitzt einen pH-Wert von kleiner als 10.

Durch Halogenierung des Farbstoffs und Verringerung des pH-Werts gegenüber Mitteln des Stands der Technik ist eine verbesserte Stabilität der Färbezubereitung (C) gewährleistet, wodurch die Lagerstabilität erhöht wird, und ein gleichbleibendes Färbeergebnis auch nach längerer Lagerung der Färbezubereitung (C) garantiert ist.

Das Mittel enthält als direktziehenden Farbstoff mindestens einen anionischen direktziehenden Farbstoff, ausgewählt aus Tetrabromphenolblau und Acid Red 92 (auch D&C RED No. 28 oder Phloxin B).

In einer weiteren Ausführungsform enthält die Färbezubereitung (C) mindestens eine Kombination aus mindestens einem blauen direktziehenden Farbstoff und einem roten direktziehenden Farbstoff, wobei das Gewichtsverhältnis zwischen der Summe aller blauen direktziehenden Farbstoffe und der Summe aller roten direktziehenden Farbstoffe einen Wert von größer oder gleich 1 besitzt. Dadurch ist es möglich, unerwünschte Farbverschiebungen in Richtung rosa-/rosé-farbene Nuancen zu vermieden.

Erfindungsgemäß bevorzugte Farbstoffkombinationen sind solche, die mindestens die Kombination aus Tetrabromphenolblau und Acid Red 92 enthalten.

Erfindungsgemäß besonders bevorzugt sind Mittel, deren Färbezubereitung (C) als halogenierten, direktziehenden Farbstoff mindestens eine Farbstoffkombination aus Tetrabromphenolblau und Acid Red 92 enthält.

Erfindungsgemäß bevorzugte Mittel sind dadurch gekennzeichnet, dass das Gesamtgewicht aller blauen direktziehenden Farbstoffe größer als das Gesamtgewicht aller roten direktziehenden Farbstoffe ist.

Bevorzugte Mittel sind daher dadurch gekennzeichnet, das Gewichtsverhältnis zwischen der Summe aller blauen direktziehenden Farbstoffe und der Summe aller roten direktziehenden Farbstoffe einen Wert von 1 bis 100, bevorzugt von 1,5 bis 10 und besonders bevorzugt von 2 bis 4 besitzt.

Es kann erfindungsgemäß weiterhin bevorzugt sein, wenn das Mittel weitere direktziehende Farbstoffe enthält. Besonders bevorzugt enthält das Mittel als weiteren direktziehenden Farbstoff einen gelben und/oder orangen Farbstoff.

Dies ist insbesondere dann vorteilhaft, wenn unerwünschte rötliche Farbverschiebungen beim Blondierprozess auftreten.

Bevorzugt werden die gelben Farbstoffe ausgewählt aus geeigneten gelben Nitrofarbstoffe, wie1,2-Diamino-4-nitrobenzol (C.I. 76,020), 1-[(2-Hydroxyethyl)amino]-2-nitrobenzol (HC Yellow 2), 1-(2-Hydroxyethoxy)-2-[(2-hydroxyethyl)amino]-5-nitrobenzol (HC Yellow 4), 1-Amino-2-[(2-hydroxyethyl)amino]-5-nitrobenzol (HC Yellow 5), 2-[Di(2-hydroxyethyl)amino]-5-nitrophenol, 2-[(2-Hydroxyethyl)amino]-1-methoxy-5-nitrobenzol, 2-Amino-3-nitrophenol, 2-Amino-4-nitrophenol, 1-Amino-2-methyl-6-nitrobenzol, 1-(2-Hydroxyethoxy)-3-methylamino-4-nitrobenzol, 2,3-(Dihydroxy-propoxy)-3-methylamino-4-nitrobenzol, 3-[(2-Aminoethyl)amino]-1-methoxy-4-nitrobenzol-hydrochlorid (HC Yellow 9), 2-[(2-Hydroxyethyl)amino]-5-nitrophenol (HC Yellow 11), 1-[(2'-Ureidoethyl)amino]-4-nitrobenzol, 1-Amino-4-[(2-aminoethyl)amino]-5-methyl-2-nitrobenzol, 4-[(2-Hydroxyethyl)amino]-3-nitro-1-methylbenzol, 4-[(2-Hydroxyethyl)amino]-3-nitrobenzonitril (HC Yellow 14), 4-[(2-Hydroxyethyl)amino]-3-nitrobenzamid (HC Yellow 15) 3-[(2-Hydroxyethyl)amino]-4-methyl-1-nitrobenzol, 4-Chlor-3-[(2-hydroxyethyl)amino]-1-nitrobenzol.

Geeignete gelbe oder orange Chinonfarbstoffe sind insbesondere 2-[(2-Aminoethyl)amino]-9,10-anthrachinon (HC Orange 5) und 2-Hydroxy-1,4-naphthochinon (C.I. 75,480, Natural Orange 6). Auch neutrale, gelbe oder orange Azofarbstoffe können erfindungsgemäß vorteilhaft eingesetzt werden, insbesondere 4-[(4-Aminophenyl)azo]-1-[di(2-hydroxyethyl)amino]-3-methylbenzol (HC Yellow 7), 2,6-Diamino-3-[(pyridin-3-yl)azo]pyridin, 2-{[4-(Acetylamino)phenyl]azo}-4-methylphenol (C.I. 11855; Disperse Yellow 3), 4-[(4-Nitrophenyl)azo]-anilin (C.I. 11,005; Disperse Orange 3).

Als gelbe oder orange, anionische direktziehende Farbstoffe eignen sich insbesondere 6-Hydroxy-5-[(4-sulfophenyl)azo]-2-naphthalinsulfonsäuredinatriumsalz (C.I. 15,985; Food Yellow No. 3; FD&C Yellow No. 6), 2,4-Dinitro-1-naphthol-7-sulfonsäure-dinatriumsalz (C.I. 10,316; Acid Yellow 1; Food Yellow No. 1), 2-(Indan-1,3-dion-2-yl)chinolin-x,x-sulfonsäure (Gemisch aus Mono- und Disulfonsäure) (C.I. 47,005; D&C Yellow No. 10; Food Yellow No. 13; Acid Yellow 3, Yellow 10), 4-((4-Amino-3-sulfophenyl)azo)benzolsulfonsäure-dinatriumsalz (C.I. 13,015, Acid Yellow 9), 5-Hydroxy-1-(4-sulfophenyl)-4-[(4-sulfophenyl)azo]pyrazol-3-carbonsäure-trinatriumsalz (C.I. 19,140; Food Yellow No. 4; Acid Yellow 23), 3-[(4-Phenylamino)phenyl]azobenzolsulfonsäure-natriumsalz (C.I. 13,065; Ki406; Acid Yellow 36), 9-(2-Carboxyphenyl)-6-hydroxy-3H-xanthen-3-on (C.I. 45,350; Acid Yellow 73; D&C Yellow No. 8), 5-[(2,4-Dinitrophenyl)amino]-2-phenylamino-benzolsulfonsäure-natriumsalz (C.I. 10,385; Acid Orange 3), 4-[(2,4-Dihydroxyphenyl)azo]benzolsulfonsäure-natriumsalz (C.I. 14,270; Acid Orange 6), 4-[(2-Hydroxynaphth-1-yl)azo]-benzolsulfonsäure-natriumsalz (C.I. 15,510; Acid Orange 7), 4-[(2,4-Dihydroxy-3-[(2,4-dimethylphenyl)azo]-phenyl)azo]benzolsulfonsäure-natriumsalz (C.I. 20,170; Acid Orange 24).

Als kationische direktziehende Farbstoffe eignen sich insbesondere 3-[(4-Amino-2,5-dimethoxyphenyl)azo]-N,N,N-trimethylbenzolaminiumchlorid (C.I. 12,605, Basic Orange 69), Di[4-(dimethylamino)phenyl]iminomethan-hydrochlorid (C.I. 41,000; Basic Yellow 2), 2-[2-((2,4-Dimethoxyphenyl)amino)ethenyl]-1,3,3-trimethyl-3H-indol-1-ium-chlorid (C.I. 48,055; Basic Yellow 11), 3-Methyl-1-phenyl-4-[(3-(trimethylammonio)phenyl)azo]pyrazol-5-on-chlorid (C.I. 12,719; Basic Yellow 57). Bevorzugte gelbe oder orange, kationische direktziehende Farbstoffe sind Basic Yellow 87 und Basic Orange 31.

Besonders bevorzugte gelbe oder orange, nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Yellow 13, HC Orange 1 und Disperse Orange 3, insbesondere HC Yellow 13.

Es ist erfindungsgemäß besonders vorteilhaft, wenn im anwendungsbereiten Mittel die Gesamtmenge an direktziehenden Farbstoffen 0,0001 bis 2,5 Gew.-%, bezogen auf das Gesamtgewicht des anwendungsbereiten Mittels, beträgt.

Erfindungsgemäße Mittel sind dadurch gekennzeichnet, dass die Färbezubereitung (C) einen neutralen bis schwach alkalischen pH-Wert besitzen. Unter schwach alkalisch ist hierbei ein pH-Wert von kleiner 10 zu verstehen. Dies führt zu einer erheblichen Verbesserung der Lagerstabilität der Färbezubereitung (C).

Eine weitere bevorzugte Ausführungsform der vorliegenden Erfindung besteht darin, dass die Färbezubereitung (C) einen pH-Wert zwischen 5,0 und 10,0, bevorzugt zwischen 5,0 und 9,6, besitzt. Bei den pH-Werten im Sinne der vorliegenden Erfindung handelt es sich um pH-Werte, die bei einer Temperatur von 22 °C gemessen werden.

Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak.

Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Erfindungsgemäß einsetzbare, organische Alkalisierungsmittel werden bevorzugt ausgewählt aus Alkanolaminen aus primären, sekundären oder tertiären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methylpropanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethyl-ethanolamin, Methylglucamin, Triethanolamin, Diethanolamin und Triisopropanolamin. Ein insbesondere bevorzugtes Alkanolamin ist Monoethanolamin. Erfindungsgemäß geeignete, basische Aminosäuren sind insbesondere Arginin, Lysin und Ornithin. Erfindungsgemäße, anorganische Alkalisierungsmittel werden bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat.

Die erfindungsgemäßen Zusammensetzungen enthalten die Alkalisierungsmittel bevorzugt in Mengen von 0,2 bis 25 Gew.-%, insbesondere 0,5 bis 10 Gew.-%.

Die Anmelderin konnte weiter finden, dass der Zusatz eines Komplexbildners in der Färbezubereitung (C) einen erheblichen Zusatzeffekt zur Stabilität der Färbezubereitung liefert und damit zum Erhalt der Färbeleistung der Mittel beiträgt.

Die vorliegende Erfindung ist daher dadurch gekennzeichnet, dass im Mittel die Färbezubereitung (C) zusätzlich mindestens einen Komplexbildner enthält. Komplexbildner wurden bereits voranstehend beschrieben.

Bevorzugt enthält die Färbezubereitung (C) als Komplexbildner mindestens eine Verbindung, die ausgewählt ist aus der Gruppe, die gebildet wird aus Ethylendiamintetraessigsäure (EDTA), Diethylentriaminpentaessigsäure (DTPA), Ethylendiamindibernsteinsäure (EDDS), Nitrilotriessigsäure (NTA), Ethylendiamindiglutarsäure (EDGA), 2-Hydroxypropylendiamindibernsteinsäure (HPDS), Glycinamid-N,N'-dibernsteinsäure (GADS), Ethylendiamin-N-N'-diglutarsäure (EDDG), 2-Hydroxypropylendiamin-N-N'-dibernsteinsäure (HPDDS), Diaminoalkyldi-(sulfo-bernsteinsäure) (DDS), Ethylendicysteinsäure (EDC), Ethylendiamin-N-N'-bis(orthohydroxyphenyl)essigsäure (EDDHA), 1-Hydroxyethan-1,1-diphosphonsäure (HEDP), Ethylendiamintetra(methylenphosphonsäure) (EDTMP), Diethylentriaminpenta(methylenphosphonsäure) (DTPMP), 2-Phosphonobutan-1,2,4-tricarbonsäure, sowie deren physiologisch verträglichen Salzen.

Eine bevorzugte Ausführungsform ist dadurch gekennzeichnet, dass die Färbezubereitung (C) als Komplexbildner mindestens 1-Hydroxyethan-1,1-diphosphonsäure und/oder eines deren physiologisch verträglichen Salze enthält.

Die Färbezubereitung (C) enthält die erfindungsgemäßen Komplexbildner bevorzugt in einer Menge von 0,1 bis 10 Gew.-%, bevorzugt 0,2 bis 5 Gew.-%, bezogen auf das Gesamtgewicht der Färbezubereitung (C).

Vorzugsweise sind die Oxidationsmittelzubereitung (A) und die mattierende Färbezubereitung (C) als fließfähige Zubereitungen konfektioniert. Dazu wird den fließfähigen Zubereitungen (A) und (C) weiterhin ein Emulgator bzw. ein Tensid zugesetzt, wobei oberflächenaktive Substanzen je nach Anwendungsgebiet als Tenside oder als Emulgatoren bezeichnet werden und aus anionischen, kationischen, zwitterionischen, amphoteren und nichtionischen Tensiden und Emulgatoren ausgewählt sind.

Bevorzugte anionische Tenside sind lineare und verzweigte Fettsäuren mit 8 bis 30 C-Atomen (Seifen), Alkylsulfate, Alkylethersulfate und Ethercarbonsäuren Formel RO(CH₂CH₂O)ₓCH₂COOH, in der R eine lineare Alkylgruppe mit 8 bis 30 C-Atomen und x = 0 oder 1 bis 16 ist.

Besonders geeignete zwitterionische Tenside sind die sogenannten Betaine wie die N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Erfindungsgemäß geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte amphotere Tenside sind das N-Kokosalkylaminopropionat, das Kokosacylaminoethylaminopropionat und das C₁₂-C₁₈-Acylsarcosin.

Als nichtionische Tenside eignen sich insbesondere C₈-C₂₂-Alkylmono- und oligoglycoside und deren ethoxylierte Analoga sowie die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure. Zubereitungen mit hervorragenden Eigenschaften werden ebenfalls erhalten, wenn sie als nichtionische Tenside Fettsäureester von ethoxyliertem Glycerin enthalten.

Die anionischen, nichtionischen, zwitterionischen oder amphoteren Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Amidoamine (z. B. Stearamidopropyldimethylamin: Tegoamid® S 18). Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen (z. B. Warenzeichen Stepantex®, Dehyquart® und Armocare®). Weitere erfindungsgemäß verwendbare kationische Tenside stellen die quaternisierten Proteinhydrolysate dar.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer bevorzugten Ausführungsform können nicht-ionische, zwitterionische und/oder amphotere Tenside sowie deren Mischungen bevorzugt sein.

Das anwendungsbereite kann weitere Zusatz- und Hilfsstoffe enthalten, die der Fachmann nach bedarf auszuwählen und in oder mehrere Zubereitungen (A), (B) und (C) einzuarbeiten weiß. Weitere Wirk-, Hilfs- und Zusatzstoffe sind beispielsweise
- nichtionische Polymere wie Vinylpyrrolidinon/Vinylacrylat-Copolymere, Polyvinylpyrrolidinon und Vinylpyrrolidinon/Vinylacetat-Copolymere und Polysiloxane,
- zwitterionische und amphotere Polymere wie Acrylamidopropyltrimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie Polyacrylsäuren, vernetzte Polyacrylsäuren, Vinylacetat/ Crotonsäure-Copolymere, Vinylpyrrolidinon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert-Butyl-acrylamid-Terpolymere,
- Verdickungsmittel, wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, Stärke-Fraktionen und Derivate, wie Amylose, Amylopektin und Dextrine, Tone wie Bentonit, oder vollsynthetische Hydrokolloide, wie Polyvinylalkohol,
- Strukturanten wie Maleinsäure und Milchsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle,
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere Benzophenone, Zimtsäure-Derivate und Triazine,
- Wirkstoffe wie Allantoin, Pyrrolidoncarbonsäuren und deren Salze sowie Bisabolol,
- Cholesterin,
- Konsistenzgeber wie Zuckerester, Polyolester oder Polyolalkylether,
- weitere Fette und Wachse wie Fettalkohole, Bienenwachs, Montanwachs und Paraffine,
- Fettsäurealkanolamide,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Treibmittel wie Propan-Butan-Gemische, N₂O, Dimethylether, CO₂ und Luft,
- Antioxidantien.

Die Auswahl dieser weiteren Stoffe wird der Fachmann gemäß der gewünschten Eigenschaften der Mittel treffen.

Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Es ist bevorzugt, die einzelnen Zubereitungen (A), (B) und (C) in möglichst kurzer zeitlicher Abfolge, möglichst innerhalb weniger als 5 min, miteinander zu vermischen und das anwendungsbereite Mittel anschließend zeitnah, möglichst innerhalb von weniger als 30 min, auf das Haar zu applizieren.

Die gebrauchsfertigen Mittel aus Oxidationsmittelzubereitung (A), Blondierpulver (B) und Färbezubereitung (C) besitzen bevorzugt einen Misch-pH-Wert im Bereich von 6 bis 12. Besonders bevorzugt ist die Anwendung im alkalischen Milieu. Die Anwendungstemperaturen können in einem Bereich zwischen 15 und 40 °C liegen. Nach einer Einwirkungszeit von 2 bis 60 min, bevorzugt 5 bis 45 min wird das Blondiermittel durch Ausspülen von dem Haar entfernt. Das Nachwaschen mit einem Shampoo entfällt, wenn ein stark tensidhaltiger Träger verwendet wurde.

Im Rahmen dieses Gegenstandes der vorliegenden Erfindung gelten die oben getroffenen Aussagen analog.

Ein weiterer Gegenstand der vorliegenden Erfindung ist eine Mehrkomponentenverpackungseinheit (Kit-of-Parts), welche mindestens drei getrennt voneinander konfektionierte Container umfasst und welche dadurch gekennzeichnet ist,
i. dass ein erster Container (I) mindestens eine Oxidationszubereitung (A), enthaltend in einem flüssigen, kosmetischen Träger mindestens ein Oxidationsmittel, enthält,
ii. dass ein zweiter Container (II) mindestens eine Blondierpulver (B), enthaltend mindestens einen Bleichkraftverstärker und mindestens ein festes Alkalisierungsmittel, enthält, und
iii. dass ein dritter Container (III) mindestens eine Färbezubereitung (C), enthaltend in einem flüssigen, kosmetischen Träger mindestens einen halogenierten, direktziehenden Farbstoff, ausgewählt aus der Gruppe, die gebildet wird aus Tetrabromphenolblau und Acid Red 92 und zusätzlich mindestens einen Komplexbildner, bei einem pH-Wert von kleiner 10, enthält,
wobei die Gesamtmenge an direktziehenden Farbstoffen 0,0001 bis 2,5 Gew.-%, bezogen auf das Gesamtgewicht des anwendungsbereiten Mittels, beträgt, und
wobei sich die Angaben "fließfähig", "fest" und "flüssig" auf eine Temperatur im Bereich zwischen 15 und 40 °C beziehen.

Hinsichtlich der bevorzugten Ausführungsführungsformen der Oxidationsmittelzubereitung (A), des Blondierpulver (B) und der Färbezubereitung (C) gelten die obigen Ausführungen.

Unter einem Container ist im Rahmen der Erfindung ein Behältnis in Form einer Dose, eines Fläschchens, einer Tüte oder eines Sachets zu verstehen. Dabei können unterschiedliche Materialien, insbesondere Kunststoffe, eingesetzt werden. Die Container können dabei, wo Bedarf danach besteht, wiederverschließbar sein.

Die Mischung der Zubereitungen (A), (B) und (C) vor der Anwendung führt zu einem erfindungsgemäßen, anwendungsbereiten Mittel.

Weiterhin kann es erfindungsgemäß besonders vorteilhaft sein, wenn das genannte Kit-of-Parts mindestens ein weiteres Haarbehandlungsmittel in einem getrennten Container enthält, insbesondere ein Konditioniermittel. Darüber hinaus kann die Verpackungseinheit Applikationshilfen, wie Kämme, Bürsten oder Pinsel, persönliche Schutzkleidung, insbesondere Ein-Weg-Handschuhe, sowie gegebenenfalls eine Gebrauchsanleitung umfassen.

Ein weiterer Gegenstand der vorliegenden Anmeldung ist schließlich die Verwendung einer Mehrkomponentenverpackungseinheit gemäß dem vorangegangenen Erfindungsgegenstand zur Stabilisierung der Farbstoffe und zur Erzielung von gleichbleibenden Färbeergebnissen während der Aufhellung und/oder Blondierung menschlicher Haare.

Im Rahmen dieses Gegenstandes der vorliegenden Erfindung gelten die oben getroffenen Aussagen analog.

Die folgenden Beispiele sollen den Gegenstand der vorliegenden Erfindung näher erläutern ohne diesen in irgendeiner Weise zu beschränken.

### Beispiele

Die Mengenangaben in den Beispielen verstehen sich, soweit nichts anderes vermerkt ist, in Gewichtsprozent. Es wurden die folgenden Zubereitungen hergestellt:

### 1 Färbezubereitungen (C)

| **Rohstoff** | **C1** | **C2** | **C3** |
|---|---|---|---|
| Ammonium Carbomer, 1% | 15,00 | 15,00 | 15,00 |
| Lanette E, Pulver [1] | 0,70 | 0,70 | 0,70 |
| Texapon NSF, 27% [2] | 4,40 | 4,40 | 4,40 |
| Kalium Oleat, 12,5% | 3,00 | 3,00 | 3,00 |
| Cutina GMS SE [3] | 2,00 | 2,00 | 2,00 |
| Cutina AGS [4] | 2,00 | 2,00 | 2,00 |
| Eutanol G [5] | 2,00 | 2,00 | 2,00 |
| Hvdrenol D [6] | 12,00 | 12,00 | 12,00 |
| Phospholipid EFA [7] | 0,10 | 0,10 | 0,10 |
| Eumulqin B 2 [8] | 3,00 | 3,00 | 3,00 |
| Titandioxid | 0,50 | 0,50 | 0,50 |
| Merquat Plus 3330 [9] | 1,50 | 1,50 | 1,50 |
| Styleze CC-10 [10] | -- | 1,00 | 1,00 |
| Citronensäure | -- | 0,50 | 0,25 |
| Phenoxyethanol | -- | 1,00 | -- |
| Tetrabromphenolblau | 0,12 | 0,12 | 0,12 |
| Acid Red 92 | 0,04 | 0,04 | 0,04 |
| HEDP, 60% | -- | -- | 0,40 |
| Na₄-EDTA, Pulver, 87% | 0,20 | 0,20 | 0,20 |
| Ascorbinsäure | 0,04 | -- | -- |
| Natriumchlorid | -- | -- | 0,40 |
| Puricare LS 9658 [11] | 1,00 | 1,00 | 1,00 |
| Crodarom Rock Crvstal [12] | 0,10 | 0,10 | 0,10 |
| Monoethanolamin | 4,00 | -- | -- |
| Ammoniak, 25 % | 2,00 | 2,00 | 2,00 |
| Parfüm | qs | qs | qs |
| Wasser, entsalzt | ad 100 | | |

Zubereitung C1 besitzt einen pH-Wert von 11 und führt damit zu keinem erfindungsgemäßen Mittel; die Zubereitungen C2 und C3 besitzen jeweils einen pH-Wert von 9,5 und sind daher erfindungsgemäß.

Alle Zubereitungen wurden bei 60°C gelagert und ihre Stabilität hinsichtlich Färbeleistung wurde nach 1 Woche sowie nach 2 Wochen untersucht.

### 2 Oxidationsmittelzubereitung (A)

| **Rohstoff** | **Menge [Gew.-%]** |
|---|---|
| NaBenzoat | 0,04 |
| Dipicolinsäure | 0,10 |
| Dinatriumpyrophosphat | 0,10 |
| Kaliumhydroxid 50% | 0,19 |
| 1,2-Propandiol | 1,50 |
| HEDP, 60% | 0,25 |
| Paraffinum Liquidum | 0,30 |
| Genamin STAC [13] | 0,39 |
| Cetearyl Alcohol | 3,40 |
| Eumulgin B 2 | 1,00 |
| Wasserstoffperoxid, 50 % | 24,30 |
| Wasser | ad 100 |

### 3 Blondierpulver (B)

| **Rohstoff** | **Menge [Gew.-%]** |
|---|---|
| Natriummetasilikat | 11,8 |
| Portil N [14] | 23,8 |
| Magnesiumcarbonat | 22,9 |
| Natriumhexametaphosphat | 0,2 |
| Rohaqit S hv [15] | 2,0 |
| Na₂EDTA | 0,6 |
| Cekol 50000 [16] | 1,0 |
| Celquat L-200 [17] | 0,3 |
| Kieselsäure, pyrogen | 0,4 |
| Glycin | 0,6 |
| Natriumperoxodisulfat | 14,5 |
| Ammoniumperoxodisulfat + 0,5 % Kieselsäure | 10,0 |
| Dow Corning 1403 Fluid [18] | 1,5 |
| Paraffinum Liquidum | 9,7 |
| Pigments, Parfum | 0,7 |

### 4 Blondierung

Unmittelbar vor der Anwendung auf den Fasern wurden 70 g Oxidationsmittelzubereitung (A) und 35 g Blondierpulver (B) mit jeweils 15 g der gegebenenfalls gelagerten Färbezubereitungen (C) vermischt.

Die resultierende Anwendungszubereitung wurde auf Strähnen von Yak-Haar in einem Flottenverhältnis von 4 zu 1 aufgetragen und 45 min bei 35 °C auf den Strähnen belassen. Anschließend wurden die Strähnen mit Wasser gründlich gespült und mit Hilfe eines Föns getrocknet.

Die so gefärbten Strähnen wurden mit Hilfe des Lab-Systems vermessen und auf ihre Farbstärke hin untersucht. Die Farbstärke nach Lagerung wurde dabei in das Verhältnis zur Farbstärke derselben Mittel ohne zuvor gelagerte Färbezubereitung gesetzt.

Die gemessenen Farbstärken sind in nachfolgender Tabelle zusammengestellt.

| Mittel | Lagerung der Färbezubereitung C | Farbstärke gegenüber ungelagerter Zubereitung C |
|---|---|---|
| A + B + C1 (pH 11) (Vergleich) | 60°C, 1 Woche | 64,9 % |
| A + B + C1 (pH 11) (Vergleich) | 60°C, 2 Wochen | 60,7 % |
| A + B + C2 (pH 9,5) (erfindungsgemäß) | 60°C, 1 Woche | 75,4 % |
| A + B + C2 (pH 9,5) (erfindungsgemäß) | 60°C, 2 Wochen | 73,1 % |
| A + B + C3 (pH 9,5) (erfindungsgemäß) | 60°C, 1 Woche | 104,8 % |
| A + B + C3 (pH 9,5) (erfindungsgemäß) | 60°C, 2 Wochen | 99,3 % |

Es ist offensichtlich, dass die erfindungsgemäßen Mittel eine verbesserte Stabilität hinsichtlich der Farbstärke aufweisen. Das mit Färbezubereitung C3 hergestellte Mittel zeigt nahezu kompletten Erhalt der Farbstärke.

### 5 Verzeichnis der eingesetzten Handelsprodukte

[1] C₁₆-C₁₈-Fettalkoholsulfat, Natrium-Salz (INCI-Bezeichnung: Sodium Cetearyl Sulfate) (Cognis)
[2] C₁₂-Fettalkoholsulfat, ethoxyliert (2 EO) Natrium-Salz (INCI-Bezeichnung: Sodium Laureth Sulfate) (Cognis)
[3] Stearinsäureglycerylester (INCI-Bezeichnung: Glyceryl Stearate) (Cognis)
[4] Ethylenglycoldistearylester (INCI-Bezeichnung: Glycol Distearate) (Cognis)
[5] 2-Octyldodecanol (INCI-Bezeichnung: Octyldodecanol) (Cognis)
[6] C₁₆-C₁₈-Fettalkohol (INCI-Bezeichnung: Cetearyl alcohol) (Cognis)
[7] zwitterionisches Phospholid (INCI-Bezeichnung: Linoleamidopropyl PG-dimonium chloride phosphate) (Uniqema)
[8] C₁₆-C₁₈-Fettalkohol, ethoxyliert (20 EO) (INCI-Bezeichnung: Ceteareth-20) (Cognis)
[9] Terpolymer aus Acylsäure, Acrylamid, Diallyldimethylammoniumchlorid (12 EO) (INCI-Bezeichnung: Polyquaternium-39) (Ondeo Nalco)
[10] Copolymer aus VP & N,N-(Dimethylamino)propylacrylamid (INCI-Bezeichnung: VP/DMAPA acrylates Copolymer) (ISP)
[11] Moringa Oleifera Samen Extrakt (INCI-Bezeichnung: Aqua, Glycerin Meringa Pterygosperma Seed Extract) (Cognis)
[12] Quartzextrakt (INCI-Bezeichnung: Aqua, Propylene Glycol, Quartz) (Crodarom)
[13] Trimethylstearylammoniumchlorid (INCI-Bezeichnung: Steartrimoniumchloride) (Clariant)
[14] Natriumsilicat (INCI-Bezeichnung: Sodium Silicate) (Cognis)
[15] Copolymer aus Methacrylsäuremethylester und Methacrylsäure (INCI-Bezeichnung: Acrylates Copolymer) (Evonik)
[16] Carboxymethylcellulose Natriumsalz (INCI-Bezeichnung: Cellulose Gum) (Kelco)
[17] quaternisierte Cellulose (INCI-Bezeichnung: Polyquaternium-4) (National Starch)
[18] Silikonzubereitung (INCI-Bezeichnung: Dimethicone, Dimethiconol) (Dow Corning)

## Patentansprüche

1. Verfahren, bei dem ein fließfähiges Mittel zur Aufhellung und Färbung menschlicher Haare durch Vermischen hergestellt wird, auf menschliches Haar aufgetragen wird, das Mittel für einen Zeitraum von 2 bis 60 min im Haar belassen wird, und das Haar anschließend mit Wasser und/oder einem handelsüblichen Shampoo gespült wird, **dadurch gekennzeichnet, dass** das Mittel durch Vermischen von
i. mindestens einer Oxidationsmittelzubereitung (A), enthaltend in einem flüssigen, kosmetischen Träger mindestens ein Oxidationsmittel,
ii. mindestens einem Blondierpulver (B), enthaltend mindestens einen Bleichkraftverstärker und mindestens ein festes Alkalisierungsmittel, und
iii. mindestens einer Färbezubereitung (C), enthaltend in einem flüssigen, kosmetischen Träger mindestens einen halogenierten, direktziehenden Farbstoff, ausgewählt aus der Gruppe, die gebildet wird aus Tetrabromphenolblau und Acid Red 92, und zusätzlich mindestens einen Komplexbildner, bei einem pH-Wert von kleiner 10, hergestellt wird,
**dadurch gekennzeichnet, dass** die Gesamtmenge an direktziehenden Farbstoffen 0,0001 bis 2,5 Gew.-%, bezogen auf das Gesamtgewicht des anwendungsbereiten Mittels, beträgt, wobei sich die Angaben "fließfähig", "fest" und "flüssig" auf eine Temperatur im Bereich zwischen 15 und 40 °C beziehen.

2. Verfahren nach Anspruch 1, **dadurch gekennzeichnet, dass** die Färbezubereitung (C) als halogenierten, direktziehenden Farbstoff mindestens eine Kombination aus Tetrabromphenolblau und Acid Red 92 enthält.

3. Verfahren einem der Ansprüche 1 bis 2, **dadurch gekennzeichnet, dass** die Färbezubereitung (C) einen pH-Wert zwischen 5 bis 9,6 besitzt.

4. Verfahren nach einem der Ansprüche 1 bis 3, **dadurch gekennzeichnet, dass** die Färbezubereitung (C) als Komplexbildner mindestens 1-Hydroxyethan-1,1-diphosphonsäure und/oder eines deren physiologisch verträglichen Salze enthält.

5. Verfahren nach einem der Ansprüche 1 bis 4, **dadurch gekennzeichnet, dass** die Blondierzubereitung (B) als Bleichkraftverstärker mindestens ein anorganisches Salz, ausgewählt aus Peroxodisulfaten, enthält.

6. Verfahren nach einem der Ansprüche 1 bis 5, **dadurch gekennzeichnet, dass** das Mittel für einen Zeitraum von 5 bis 45 min im Haar belassen wird.

7. Mehrkomponentenverpackungseinheit (Kit-of-Parts), umfassend mindestens drei getrennt voneinander konfektionierte Container, **dadurch gekennzeichnet,**
i. **dass** ein erster Container (I) mindestens eine Oxidationszubereitung (A), enthaltend in einem flüssigen, kosmetischen Träger mindestens ein Oxidationsmittel, enthält,
ii. **dass** ein zweiter Container (II) mindestens eine Blondierpulver (B), enthaltend mindestens einen Bleichkraftverstärker und mindestens ein festes Alkalisierungsmittel, enthält, und
iii. **dass** ein dritter Container (III) mindestens eine Färbezubereitung (C), enthaltend in einem flüssigen°, kosmetischen Träger mindestens einen halogenierten, direktziehenden Farbstoff, ausgewählt aus der Gruppe, die gebildet wird aus Tetrabromphenolblau und Acid Red 92 und zusätzlich mindestens einen Komplexbildner, bei einem pH-Wert von kleiner 10, enthält,
wobei die Gesamtmenge an direktziehenden Farbstoffen 0,0001 bis 2,5 Gew.-%, bezogen auf das Gesamtgewicht des anwendungsbereiten Mittels, beträgt, und
wobei sich die Angaben "fließfähig", "fest" und "flüssig" auf eine Temperatur im Bereich zwischen 15 und 40 °C beziehen.

8. Verwendung einer Mehrkomponentenverpackungseinheit gemäß Anspruch 7 zur Stabilisierung der Farbstoffe und zur Erzielung von gleichbleibenden Färbeergebnissen während der Aufhellung und/oder Blondierung menschlicher Haare.

## Claims

1. A method in which a flowable agent for lightening and dyeing human hair is prepared by mixing and applied to human hair, the agent is left in the hair for a period of from 2 to 60 min, and then the hair is rinsed with water and/or a commercially available shampoo, **characterized in that** the agent is prepared by mixing
i. at least one oxidizing agent preparation (A) containing at least one oxidizing agent in a liquid cosmetic carrier,
ii. at least one bleaching powder (B) containing at least one bleach booster and at least one solid alkalizing agent, and
iii. at least one dye preparation (C) containing, in a liquid cosmetic carrier, at least one halogenated direct dye, selected from the group consisting of tetrabromophenol blue and Acid Red 92, and additionally at least one complexing agent, at a pH of less 10, **characterized in that** the total amount of direct dyes is 0.0001 to 2.5 wt.%, based on the total weight of the ready-to-use agent, the terms "flowable," "solid" and "liquid" relating to a temperature in the range between 15 and 40 °C.

2. The method according to claim 1, **characterized in that** the dye preparation (C) contains at least a combination of tetrabromophenol blue and Acid Red 92 as a halogenated direct dye.

3. The method according to one of claims 1 to 2, **characterized in that** the dye preparation (C) has a pH between 5 and 9.6.

4. The method according to one of claims 1 to 3, **characterized in that** the dye preparation (C) contains at least 1-hydroxyethane-1,1-diphosphonic acid and/or one of the physiologically acceptable salts thereof as a complexing agent.

5. The method according to one of claims 1 to 4, **characterized in that** the bleaching preparation (B) contains at least one inorganic salt selected from peroxodisulfates as a bleach booster.

6. The method according to one of claims 1 to 5, **characterized in that** the agent is left in the hair for a period of from 5 to 45 min.

7. A multi-component packaging unit (kit-of-parts), comprising at least three containers that are manufactured separately from one another, **characterized in that**
i. a first container (I) contains at least one oxidation preparation (A) containing at least one oxidizing agent in a liquid cosmetic carrier,
ii. a second container (II) contains at least one bleaching powder (B) containing at least one bleach booster and at least one solid alkalizing agent, and
iii. a third container (III) contains at least one dye preparation (C) containing, in a liquid cosmetic carrier, at least one halogenated direct dye, selected from the group consisting of tetrabromophenol blue and Acid Red 92, and additionally at least one complexing agent, at a pH of less 10,
the total amount of direct dyes being 0.0001 to 2.5 wt.%, based on the total weight of the ready-to-use agent, and
the terms "flowable," "solid" and "liquid" relating to a temperature in the range between 15 and 40 °C.

8. The use of a multi-component packaging unit according to claim 7 for stabilizing the dyes and for achieving consistent dyeing results during the lightening and/or bleaching of human hair.

## Revendications

1. Procédé selon lequel un agent coulant destiné à éclaircir et à colorer des cheveux humains est préparé par mélange, et est appliqué sur des cheveux humains, l'agent est laissé sur les cheveux pendant une durée de 2 à 60 minutes, puis les cheveux sont rincés avec de l'eau et/ou un shampooing disponible dans le commerce, **caractérisé en ce que** l'agent est préparé en mélangeant
i. au moins une préparation d'agent oxydant (A) contenant au moins un agent oxydant dans un support cosmétique liquide,
ii. au moins une poudre de décoloration (B) contenant au moins un renforçateur de pouvoir de blanchiment et au moins un agent alcalinisant solide, et
iii. au moins une préparation colorante (C) contenant au moins un colorant halogéné à action directe dans un support cosmétique liquide, choisi dans le groupe constitué par le bleu de tétrabromophénol et le Rouge acide 92, et en outre au moins un agent complexant, à un pH inférieur à 10,
**caractérisé en ce que** la quantité totale de colorants à action directe est de 0,0001 à 2,5 % en poids, par rapport au poids total de l'agent prêt à l'emploi, les indications
« coulant », « solide » et « liquide » se rapportant à une température dans la plage comprise entre 15 et 40 °C.

2. Procédé selon la revendication 1, **caractérisé en ce que** la préparation colorante (C) contient, en tant que colorant halogéné à action directe, au moins une combinaison de bleu de tétrabromophénol et de Rouge acide 92.

3. Procédé selon l'une des revendications 1 à 2, **caractérisé en ce que** la préparation colorante (C) présente un pH compris entre 5 et 9,6.

4. Procédé selon l'une des revendications 1 à 3, **caractérisé en ce que** la préparation colorante (C) contient, en tant qu'agent complexant, au moins de l'acide 1-hydroxyéthane-1,1-diphosphonique et/ou l'un de ses sels physiologiquement acceptables.

5. Procédé selon l'une des revendications 1 à 4, **caractérisé en ce que** la préparation de décoloration (B) contient, en tant que renforçateur de pouvoir de blanchiment, au moins un sel inorganique choisi parmi les peroxodisulfates.

6. Procédé selon l'une des revendications 1 à 5, **caractérisé en ce que** l'agent est laissé sur les cheveux pendant une durée de 5 à 45 minutes.

7. Unité de conditionnement multi-composants (kit), comprenant au moins trois récipients assemblés séparément, **caractérisée**
i. **en ce qu'**un premier récipient (I) contient au moins une préparation oxydante (A) contenant au moins un agent oxydant dans un support cosmétique liquide,
ii. **en ce qu'**un deuxième récipient (II) contient au moins une poudre de décoloration (B) contenant au moins un renforçateur de pouvoir de blanchiment et au moins un agent alcalinisant solide, et
iii. **en ce qu'**un troisième récipient (III) contient au moins une préparation colorante (C) contenant au moins un colorant halogéné à action directe dans un support cosmétique liquide, choisi dans le groupe constitué par le bleu de tétrabromophénol et le Rouge acide 92, et en outre au moins un agent complexant, à un pH inférieur à 10,
la quantité totale de colorants à action directe étant de 0,0001 à 2,5 % en poids, par rapport au poids total de l'agent prêt à l'emploi, et
les indications « coulant », « solide » et « liquide » se rapportant à une température dans la plage comprise entre 15 et 40 °C.

8. Utilisation d'une unité de conditionnement multi-composants selon la revendication 7, pour stabiliser les colorants et obtenir des résultats de coloration homogènes lors de l'éclaircissement et/ou de la décoloration de cheveux humains.
